# FASCICULE DE BREVET EUROPEEN

(11) **EP 3 049 183 B1**
(45) Date de publication et mention de la délivrance du brevet: **27.01.2021**
(21) Numéro de dépôt: 14796198.1
(22) Date de dépôt: 25.09.2014
(51) Int. Cl.: C10L 3/08

(54) **RÉACTEUR DE MÉTHANATION POUR FAIRE RÉAGIR DU DIHYDROGÈNE AVEC AU MOINS UN COMPOSÉ À BASE DE CARBONE ET PRODUIRE DU MÉTHANE**
METHANISIERUNGSREAKTOR ZUR REAKTION VON DIHYDROGEN MIT MINDESTENS EINEM KOHLENSTOFFHALTIGEN VERBINDUNG UND HERSTELLUNG VON METHAN
METHANATION REACTOR FOR REACTING DIHYDROGEN WITH AT LEAST ONE CARBON-BASED COMPOUND AND PRODUCING METHANE

(30) Priorité: 26.09.2013 FR 1359314
(43) Date de publication de la demande: 03.08.2016
(73) Titulaire: ENGIE, 92400 Courbevoie (FR)
(72) Inventeur: KARA, Ylmaz, F-95600 Eaubonne (FR); MARCHAND, Bernard, F-75018 Paris (FR)
(74) Mandataire: Cornuejols, Georges
(86) Numéro de dépôt international: PCT/FR2014/052409
(87) Numéro de publication internationale: WO 2015/044599

(56) Documents cités:
- EP-A1- 0 042 519
- US-A- 2 394 680
- US-A- 3 991 096
- US-A- 4 258 006
- US-A- 5 700 432

## Description

### DOMAINE TECHNIQUE DE L'INVENTION

La présente invention vise un réacteur de méthanation pour faire réagir de l'hydrogène avec un composé à base de carbone et produire du méthane. Elle s'applique notamment à la méthanation industrielle et à la cogénération d'énergie thermique et de méthane.

### ETAT DE LA TECHNIQUE

La méthanation est un procédé industriel de conversion catalytique de l'hydrogène et du monoxyde ou du dioxyde de carbone en méthane.

Selon la nature du composé à base de carbone, la formule de la réaction de méthanation change. Cette formule est, selon le cas :

CO + 3 H₂ → CH₄ + H₂O

CO₂ + 4 H₂ → CH₄ + 2 H₂O

Afin d'optimiser les rendements de cette réaction, un lit de catalyseur est placé dans un réacteur dans lequel a lieu la réaction. Ce lit peut être fixe ou fluidisé. La réaction de méthanation étant fortement exothermique, elle entraîne des besoins importants d'évacuation de la chaleur et donc de refroidissement du réacteur. Un lit de catalyseur fluidisé permet d'homogénéiser la température de la zone réactive. Enfin, la cinétique de cette réaction aux températures habituellement mises en œuvre est élevée, nécessitant, de fait, une masse faible de catalyseur.

Dans des systèmes actuels à lits fixes, appelés « Throughwall Cooled Reactor » (traduit en français par « Réacteur refroidi à travers les parois »), un échange thermique est réalisé par des parois du réacteur refroidies par un fluide de refroidissement. Cependant les surfaces nécessitées pour réaliser l'échange thermique sont importantes et les coûts de fabrication du réacteur sont élevés.

Dans des systèmes actuels à lits fluidisés, un ou plusieurs échangeurs de chaleur sont immergés dans le lit fluidisé à l'intérieur du réacteur. On met alors en circulation dans ces échangeurs de l'eau, de la vapeur d'eau ou une huile thermique, par exemple. Les coefficients d'échange thermique entre la paroi de l'échangeur et le lit fluidisé sont très importants, de l'ordre des coefficients d'échange thermique entre un liquide et une paroi. Cependant, l'utilisation d'huiles thermiques n'est possible que jusqu'à des températures de réaction de l'ordre de 380°C à 400°C. De plus, dans ces systèmes, la taille du réacteur dépend de la taille occupée par chaque échangeur à immerger dans le lit fluidisé. Ces systèmes entraînent un coût de fabrication et une utilisation d'espace non optimisés du réacteur. Par ailleurs, l'efficacité des échanges de chaleur entre le lit et le fluide de refroidissement dépend fortement des conditions de fluidisation.

Dans des systèmes actuels à lits fixes ou à lits fluidisés, l'injection de vapeur en mélange avec l'hydrogène et le composé à base de carbone permet de limiter la forme d'un dépôt de carbone sous forme de coke sur le catalyseur dont l'une des conséquences est une désactivation prématurée du catalyseur. Enfin, les catalyseurs de méthanation étant préférentiellement constitués au moins en partie de nickel, la réaction de méthanation risque d'entrainer la formation de carbonyle, un composé hautement toxique, au contact de parois portées à une température inférieure à 260°C, ce qui complique le système de refroidissement.

On connaît les documents US2394680, US3991096, EP0042519, US5700432 et US4258006. Les documents US2394680, US3991096, EP0042519 et US5700432 enseignent l'utilisation de vapeur d'eau pour refroidir un réacteur, mais cette vapeur d'eau n'est pas surchauffée et présente donc une faible capacité d'échange thermique. Le document US4258006 enseigne un réacteur de méthanation et ne mentionne pas l'utilisation de vapeur d'eau pour refroidir la réaction à l'intérieur du réacteur.

### OBJET DE L'INVENTION

La présente invention vise à remédier à tout ou partie de ces inconvénients.

L'invention vise notamment à proposer un agencement du réacteur de méthanation comportant des moyens de refroidissements.
A cet effet, la présente invention vise, selon un premier aspect, un réacteur de méthanation pour faire réagir du dihydrogène avec au moins un composé à base de carbone et produire du méthane, selon la revendication 1.

Grâce à ces dispositions, la température de peau de l'échangeur dans le corps creux est inférieure à la température de réaction mais supérieure à 260°C. Ces dispositions permettent donc d'éviter la formation de carbonyle dans le réacteur tout en assurant une maitrise de la température de réaction en utilisant un liquide de refroidissement facile d'emploi

Dans des modes de réalisation, le réacteur objet de la présente invention comporte, en aval de la partie de l'échangeur de chaleur traversant le corps creux, un moyen de refroidissement de la vapeur surchauffée configuré pour saturer la vapeur surchauffée.

L'avantage de ces modes de réalisation est de permettre une utilisation de la vapeur ainsi refroidie à d'autres effets.

Dans des modes de réalisation, le moyen de refroidissement de la vapeur surchauffée comporte une entrée d'eau.

Ces modes de réalisation ont l'avantage de permettre à la fois de refroidir la vapeur surchauffée et de produire de la vapeur saturée pouvant être valorisée.

Dans des modes de réalisation, le réacteur objet de la présente invention comporte un capteur de température à l'intérieur du réacteur et un moyen de régulation du débit de vapeur surchauffée en fonction de la température mesurée par le capteur de température.

L'avantage de ces modes de réalisation est qu'ils permettent d'ajuster le refroidissement à l'intérieur du corps creux en fonction de la température de la réaction.

Dans des modes de réalisation, la méthanation réalisée dans le réacteur comporte la méthanation du monoxyde de carbone définie par la formule : CO + 3H₂ ⇆ CH₄ + H₂O.

Dans des modes de réalisation, la méthanation réalisée dans le réacteur comporte la méthanation du dioxyde de carbone définie par la formule : CO₂ + 4H₂ ⇆ CH₄ + 2H₂O.

Selon un deuxième aspect, la présente invention vise un procédé de méthanation pour faire réagir du dihydrogène avec au moins un composé à base de carbone et produire du méthane, selon la revendication 5.

Les avantages, buts et caractéristiques particulières de ce procédé étant similaires à ceux du réacteur de méthanation objet de la présente invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du réacteur et du procédé de méthanation pour faire réagir du dihydrogène avec au moins un composé à base de carbone et produire du méthane objets de la présente invention, en regard des dessins annexés, dans lesquels :
- la figure 1 représente, schématiquement, un mode de réalisation particulier du réacteur de méthanation objet de la présente invention et
- la figure 2 représente, sous forme d'un logigramme, des étapes d'un mode de réalisation particulier du procédé de méthanation objet de la présente invention.

### DESCRIPTION D'EXEMPLES DE REALISATION DE L'INVENTION

La présente description est donnée à titre non limitatif.

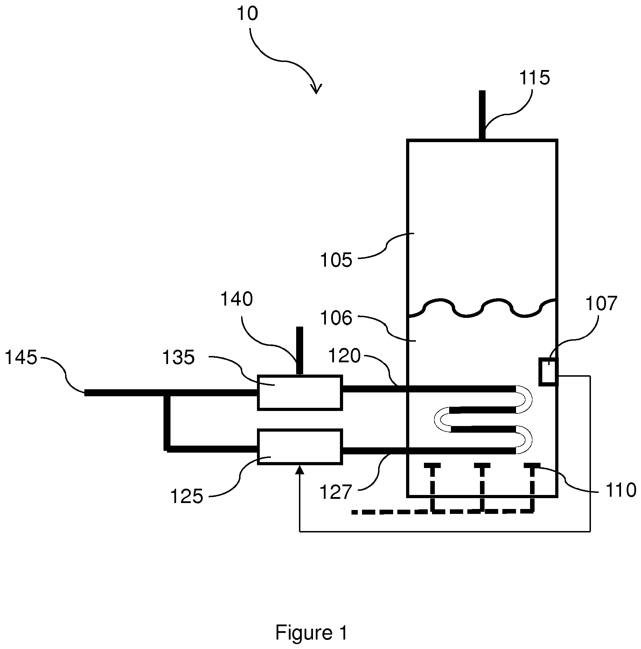

On note, dès à présent, que la figure 1 n'est pas à l'échelle.

On appelle « vapeur surchauffée » de la vapeur d'eau pour laquelle le couple pression-température est supérieur à celui du changement d'état liquide-gaz. Cette vapeur surchauffée présente une température supérieure à 260°C mais inférieure à la température de réaction à l'intérieur d'un réacteur de méthanation devant être refroidit par ladite vapeur.

On observe, sur la figure 1, un premier mode de réalisation du réacteur 10 objet de la présente invention. Ce réacteur 10 de méthanation fait réagir de l'hydrogène avec un composé à base de carbone et produit du méthane. Le réacteur 10 comporte :
- un corps creux 105 configuré pour recevoir un lit fluidisé 106 et comportant une entrée 110 du composé à base de carbone et de dihydrogène,
- une sortie de méthane et d'eau 115 produits par la réaction de méthanation,
- un échangeur 120 de chaleur traversant au moins partiellement le corps creux et au moins partiellement immergé dans le lit fluidisé 106,
- un moyen 127 d'introduction de vapeur d'eau surchauffée à l'intérieur de l'échangeur 120 de chaleur,
- un moyen de refroidissement et de saturation 135 de la vapeur surchauffée comportant une entrée d'eau 140,
- une sortie pour vapeur surchauffée 145 et
- un moyen de régulation 125 du débit de vapeur en fonction de la température. à l'intérieur du corps creux 105 mesurée par un capteur de température 107.

Le corps creux 105 est, par exemple, un cylindre de révolution métallique dont les extrémités sont closes. Ce corps creux 105 comporte une entrée non représentée configurée pour permettre l'insertion d'un lit de catalyseur fluidisé dans le corps creux 105.

Ce corps creux 105 comporte une entrée 110 du composé à base de carbone, de vapeur d'eau et de dihydrogène qui est, par exemple, une buse d'injection configurée pour injecter le composé à base de carbone dans le lit fluidisé au niveau de la base du corps creux 105.

Ce corps creux 105 comporte, en outre, une sortie de méthane et d'eau 115 produits par la réaction de méthanation. Cette sortie de méthane et d'eau 115 est, par exemple, une canalisation située sur une extrémité supérieure du corps creux 105.

Le moyen 127 d'introduction de vapeur d'eau surchauffée à l'intérieur de l'échangeur 120 de chaleur est, par exemple, un conduit permettant d'introduire de la vapeur d'eau surchauffée dans l'échangeur 120 de chaleur. Le débit de vapeur d'eau est régulé par le moyen de régulation 125.

L'échangeur de chaleur 120 est, par exemple, une canalisation tubulaire plongée dans le lit fluidisé du corps creux 105 et connecté au moyen de régulation 125 du débit de vapeur. La vapeur circule et est surchauffée dans cet échangeur 120 et réalise un échange de chaleur avec la paroi de l'échangeur 120, la paroi de l'échangeur 120 réalisant un échange de chaleur avec le lit fluidisé et abaissant, en conséquence, la température de réaction dans le corps creux 105.

Ce réacteur 10 comporte, en aval de la partie l'échangeur 120 immergée dans le lit fluidisé, et à l'extérieur du corps creux 105, un moyen de refroidissement 135 de la vapeur surchauffée comportant une entrée d'eau 140. Ce moyen de refroidissement 135 est, par exemple, un réservoir faisant la jonction entre l'eau entrée et la vapeur surchauffée provenant du l'échangeur 120.

Ce réacteur 10 comporte, en outre, une sortie pour vapeur surchauffée 145 qui est, par exemple, une canalisation permettant à une partie de la vapeur surchauffée d'être évacuée. La vapeur surchauffée conservée retourne au moyen de régulation 125 du débit de vapeur.

Ce réacteur 10 comporte, de plus, un moyen de régulation 125 du débit de vapeur surchauffée en fonction de la température à l'intérieur du corps creux. Ce moyen de régulation 125 est, par exemple, une valve commandée par un circuit électronique. Le circuit électronique régule le débit de vapeur surchauffée en fonction de la température mesurée par le capteur de température 107 pour que le débit soit une fonction croissante de la température mesurée.

Le réacteur 10 est configuré pour réaliser une réaction de méthanation comportant une méthanation du monoxyde de carbone définie par la formule : CO + 3H₂ ⇆ CH₄ + H₂O.Dans des variantes, ce réacteur 10 est configuré pour réaliser une réaction de méthanation comportant une méthanation du dioxyde de carbone définie par la formule : CO₂ + 4H₂ ⇆ CH₄ + 2H₂O.

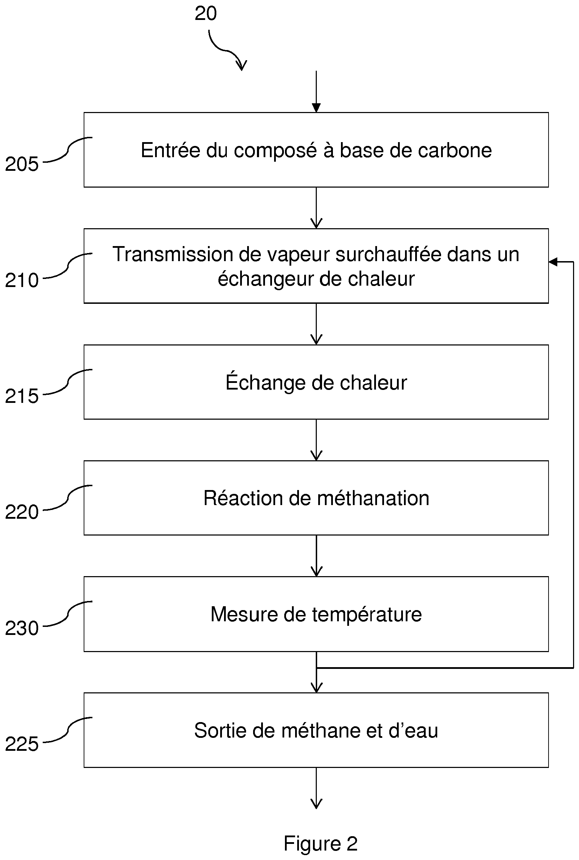

On observe, sur la figure 2, un logigramme d'étapes d'un mode de réalisation particulier du procédé 20 de méthanation pour faire réagir de l'hydrogène avec un composé à base de carbone et produire du méthane. Ce procédé 20 comporte :
- une étape 205 d'entrée du composé à base de carbone dans un corps creux configuré pour recevoir un lit fluidisé,
- une étape de 210 de transmission de vapeur surchauffée à un échangeur de chaleur traversant au moins partiellement le corps creux,
- une étape 215 d'échange de chaleur entre la vapeur et le milieu réactionnel à l'intérieur du corps creux et de surchauffe de cette vapeur,
- une étape 220 de réaction de méthanation entre de l'hydrogène et le composé à base de carbone pour produire du méthane et de l'eau,
- une étape 225 de sortie de méthane et d'eau et
- une étape 230 de mesure de température à l'intérieur du corps creux.

L'étape 205 d'entrée du composé à base de carbone dans le corps creux est réalisée, par exemple, par la mise en œuvre de buses d'injection qui injectent du monoxyde ou du dioxyde de carbone dans le corps creux.

L'étape 210 de transmission est réalisée, par exemple, par la mise en œuvre d'une turbine configurée pour pousser la vapeur surchauffée produite dans l'échangeur de chaleur.

L'étape 215 d'échange de chaleur entre la vapeur surchauffée et le milieu réactionnel a lieu par échanges de chaleur successifs entre la vapeur surchauffée et la paroi intérieure de l'échangeur et entre la paroi extérieure de l'échangeur et le lit fluidisé.

L'étape 225 de sortie de méthane et d'eau est réalisée, par exemple, par la mise en œuvre d'une canalisation située sur une surface supérieure du corps creux.

La mesure de température à l'intérieur du corps creux réalisée au cours de l'étape 230 sert à l'asservissement du débit de vapeur surchauffé introduit dans l'échangeur de chaleur au cours de l'étape 210, ce débit étant une fonction croissante de la température à l'intérieur du corps creux.

Les différentes étapes représentées en figure 2 sont réalisées en permanence et simultanément pendant le fonctionnement nominal du réacteur.

Comme on le comprend à la lecture de la description qui précède, la présente invention permet de réaliser le refroidissement d'un corps creux hôte d'une réaction de méthanation sans risque de formation de carbonyle. Le refroidissement est réalisé par la mise en circulation dans un échangeur traversant le corps creux de vapeur qui est surchauffée dans l'échangeur. Enfin, la température à l'intérieur du réacteur est asservie par introduction de vapeur selon une fonction croissante de la température mesurée dans le réacteur et la production de carbonyle peut être minimisée.

## Revendications

1. Réacteur (10) de méthanation pour faire réagir du dihydrogène avec au moins un composé à base de carbone et produire du méthane, **caractérisé en ce qu'**il comporte :
- un corps creux (105) configuré pour recevoir un lit fluidisé et comportant une entrée (110) du composé à base de carbone,
- une sortie de méthane et d'eau (115) produits par une réaction de méthanation,
- un échangeur (120) de chaleur traversant au moins partiellement le corps creux et au moins partiellement immergé dans le lit fluidisé,
- un moyen de refroidissement (135) de la vapeur surchauffée :
- configuré pour saturer la vapeur surchauffée,
- comportant un réservoir et une entrée (140) d'eau pour être injectée dans le réservoir, configuré pour mélanger l'eau et la vapeur surchauffée entrant dans le moyen de refroidissement et
- le moyen de refroidissement étant positionné en aval de la partie de l'échangeur (120) de chaleur traversant le corps creux (105),
- un moyen (127) d'introduction de vapeur d'eau surchauffée à l'intérieur de l'échangeur de chaleur.

2. Réacteur (10) selon la revendication 1, qui comporte un capteur de température (107) à l'intérieur du réacteur et un moyen de régulation (125) du débit de vapeur surchauffée en fonction de la température mesurée par le capteur de température.

3. Réacteur (10) selon l'une des revendications 1 ou 2, dans lequel la méthanation réalisée dans le réacteur comporte la méthanation du monoxyde de carbone définie par la formule : CO + 3H₂ ⇆ CH₄ + H₂O.

4. Réacteur (10) selon l'une des revendications 1 à 3, dans lequel la méthanation réalisée dans le réacteur comporte la méthanation du dioxyde de carbone définie par la formule : CO₂ + 4H₂ ⇆ CH₄ + 2H₂O.

5. Procédé (20) de méthanation pour faire réagir du dihydrogène avec au moins un composé à base de carbone et produire du méthane, **caractérisé en ce qu'**il comporte :
- une étape (205) d'entrée du composé à base de carbone dans un corps creux configuré pour recevoir un lit fluidisé,
- une étape (225) de sortie de méthane et d'eau produits par une réaction de méthanation,
- une étape (210) de transmission de vapeur surchauffée à un échangeur de chaleur traversant au moins partiellement le corps creux,
- une étape (215) d'échange de chaleur entre la vapeur et le milieu réactionnel à l'intérieur du corps creux et
- une étape de refroidissement de la vapeur surchauffée :
- pour saturer la vapeur,
- comportant une étape de mélange d'eau injectée dans un réservoir par une entrée et de vapeur surchauffée dans le réservoir et
- l'étape de refroidissement étant en aval de l'étape d'échange de chaleur à l'intérieur du corps creux.

6. Procédé selon la revendication 5, qui comporte une étape de capture de température à l'intérieur du corps creux et une étape de régulation du débit de vapeur surchauffée en fonction de la température captée.

7. Procédé selon l'une des revendications 5 ou 6, dans lequel la méthanation réalisée dans le corps creux comporte la méthanation du monoxyde de carbone définie par la formule : CO + 3H₂ ⇆ CH₄ + H₂O.

8. Procédé selon l'une des revendications 5 à 7, dans lequel la méthanation réalisée dans le corps creux comporte la méthanation du dioxyde de carbone définie par la formule : CO₂ + 4H₂ ⇆ CH₄ + 2H₂O.

## Patentansprüche

1. Methanisierungsreaktor (10) zur Reagieren des Dihydrogens mit wenigstens einem Bestandteil auf Kohlenstoffbasis und Produzieren des Methans, **dadurch gekennzeichnet, dass** er umfasst:
- einen hohlen Körper (105), der zum Aufnehmen eines Wirbelschichtbetts ausgestaltet ist und einen Eingang (110) des Bestandteils auf Kohlenstoffbasis umfasst,
- einen Methan- und Wasserausgang (115), die von einer Methanisierungsreaktion produziert sind,
- einen Wärmetauscher (120), der wenigstens teilweise den hohlen Körper und durchquert und wenigstens teilweise in dem Wirbelschichtbett eingetaucht ist,
- wenigstens einen Kühler (135) des überhitzten Dampfes:
- der zum Sättigen des überhitzten Dampfes ausgestaltet ist,
- umfassend einen Tank und einen Eingang (140) für Wasser, das in den Tank eingespritzt werden soll, der zum Vermischen des Wassers und des überhitzten Dampfes ausgestaltet ist, der in das Kühlmittel eintritt, und
- wobei das Kühlmittel dem Teil des Wärmetauschers (120) nachgeschaltet ist, der den hohlen Körper (105) durchquert,
- ein Einführmittel (127) von überhitztem Wasserdampf in das Innere des Wärmetauschers.

2. Reaktor (10) gemäß Anspruch 1, der einen Temperatursensor (107) im Innern des Reaktors und ein Regulierungsmittel (125) des überhitzten Dampfdurchsatzes in Abhängigkeit von der vom Temperatursensor gemessenen Temperatur umfasst.

3. Reaktor (10) gemäß einem der Ansprüche 1 oder 2, bei dem die in dem Reaktor durchgeführte Methanisierung die Methanisierung des Kohlenmonoxids umfasst, das von der Formel definiert ist: CO + 3H₂ ⇆ CH₄ + H₂O.

4. Reaktor (10) gemäß einem der Ansprüche 1 oder 3, bei dem die in dem Reaktor durchgeführte Methanisierung die Methanisierung des Kohlendioxids umfasst, das von der Formel definiert ist: CO₂ + 4H₂ ⇆ CH₄ + 2H₂O.

5. Methanisierungsverfahren (20) zur Reagieren des Dihydrogens mit wenigstens einem Bestandteil auf Kohlenstoffbasis und Produzieren des Methans, **dadurch gekennzeichnet, dass** es umfasst:
- einen Eingangsschritt (205) des Bestandteils auf Kohlenstoffbasis in einen hohlen Körper, der zum Aufnehmen eines Wirbelschichtbetts ausgestaltet ist,
- einen Methan- und Wasserausgangsschritt (225), die von einer Methanisierungsreaktion produziert sind,
- einen Übertragungsschritt (210) des überhitzten Dampfes auf einen Wärmetauscher, der den hohlen Körper wenigstens teilweise durchquert,
- einen Schritt (215) zum Wärmetauschen zwischen dem Dampf und dem Reaktionsmilieu im Innern des hohlen Körpers und
- einen Kühlschritt des überhitzten Dampfes:
- zum Sättigen des Dampfes,
- umfassend einen Mischschritt des Wassers, das von einem Eingang in den Tank eingespritzt ist, und des in dem Tank überhitzten Dampfes und
- wobei der Kühlschritt dem Wärmetauschschritt im Innern des hohlen Körpers nachgeschaltet ist.

6. Verfahren gemäß Anspruch 5, das einen Temperatur-Erfassungsschritt im Innern des hohlen Körpers und einen Regulierungsschritt des überhitzten Dampfdurchsatzes in Abhängigkeit von der erfassten Temperatur umfasst.

7. Verfahren gemäß einem der Ansprüche 5 oder 6, bei dem die in dem hohlen Körper durchgeführte Methanisierung die Methanisierung des Kohlenmonoxids umfasst, das von der Formel definiert ist: CO + 3H₂ ⇆ CH₄ + H₂O.

8. Verfahren gemäß einem der Ansprüche 5 oder 7, bei dem die in dem hohlen Körper durchgeführte Methanisierung die Methanisierung des Kohlendioxids umfasst, das von der Formel definiert ist: CO₂ + 4H₂ ⇆ CH₄ + 2H₂O.

## Claims

1. Methanation reactor (10) for reacting dihydrogen with at least one carbon-based compound and producing methane, comprising:
- a hollow body (105) configured to receive a fluidized bed and comprising an inlet (110) for the carbon-based compound;
- an outlet (115) for methane and water produced by a methanation reaction;
- a heat exchanger (120) extending at least partially through the hollow body and at least partially submerged in the fluidized bed;
- a means for cooling (135) the superheated vapor:
- configured to saturate the superheated vapor
- comprising a tank and an inlet of water (140) to be injected in the tank, configured to mix the water and the superheated vapor entering the means for cooling and
- the cooling means being positioned downstream of the part of the heat exchanger (120) that extends through the hollow body (105),
- a means (127) for introducing superheated water vapor into the heat exchanger.

2. Reactor (10) according to claim 1, that comprises a temperature sensor (107) inside the reactor and a means of regulating (125) the flow rate of the superheated vapor as a function of the temperature measured by the temperature sensor.

3. Reactor (10) according to one of claims 1 or 2, wherein the methanation realized in the reactor comprises the methanation of the carbon monoxide defined by the formula: CO + 3 H₂ ⇆ CH₄ + H₂O.

4. Reactor (10) according to one of claims 1 to 3, wherein the methanation realized in the reactor comprises the methanation of the carbon dioxide defined by the formula: CO₂ + 4 H₂ ⇆ CH₄ + 2 H₂O.

5. Methanation method (20) for reacting dihydrogen with at least one carbon-based compound and producing methane, comprising:
- a step (205) of inputting the carbon-based compound into a hollow body configured to receive a fluidized bed;
- a step (225) of outputting methane and water produced by a methanation reaction;
- a step (210) of transmitting superheated vapor to a heat exchanger extending at least partially through the hollow body;
- a step (215) of exchanging heat between the vapor and the reaction medium inside the hollow body and
- a step of cooling (135) the superheated vapor:
- configured to saturate the vapor,
- comprising a step of mixing water injected in a tank by an inlet and the superheated vapor in the tank and
- the cooling step being downstream of the heat exchanging step (120) through the hollow body (105).

6. Method according to claim 5, that comprises a step of capturing the temperature inside the hollow body, and a step of regulating the flow rate of the superheated vapor as a function of the temperature captured.

7. Method according to one of claims 5 or 6, wherein the methanation realized in the hollow body comprises the methanation of the carbon monoxide defined by the formula: CO + 3 H₂ ⇆ CH₄ + H₂O.

8. Method according to one of claims 5 to 7, wherein the methanation realized in the hollow body comprises the methanation of the carbon dioxide defined by the formula: CO₂ + 4 H₂ ⇆ CH₄ + 2 H₂O.
